(19) Europäisches Patentamt
European Patent Office
Office européen des brevets

(11) Veröffentlichungsnummer: **0 008 741**
**B1**

(12) **EUROPÄISCHE PATENTSCHRIFT**

(45) Veröffentlichungstag der Patentschrift: **30.09.81**

(51) Int. Cl.³: **C 07 C 45/62**, C 07 C 47/02, C 07 C 47/21, C 07 C 49/04

(21) Anmeldenummer: **79103068.7**

(22) Anmeldetag: **21.08.79**

(54) **Verfahren zur Herstellung von Carbonylverbindungen.**

(30) Priorität: **11.09.78 DE 2839474**

(43) Veröffentlichungstag der Anmeldung: **19.03.80 (Patentblatt 80/06)**

(45) Bekanntmachung des Hinweises auf die Patenterteilung: **30.09.81 Patentblatt 81/39**

(84) Benannte Vertragsstaaten: **BE CH DE FR GB IT NL**

(56) Entgegenhaltungen:
**DE - A - 2 114 211**
**DE - A1 - 2 534 747**
**FR - A - 2 224 433**

(73) Patentinhaber: **BASF Aktiengesellschaft**
**Carl-Bosch-Strasse 38**
**D-6700 Ludwigshafen (DE)**

(72) Erfinder: **Horner, Michael, Dr.**
**Pfalzgrafenstrasse 15 b**
**D-6730 Neustadt (DE)**
Erfinder: **Nissen, Axel, Dr.**
**Panoramastrasse 51**
**D-6906 Leimen (DE)**
Erfinder: **Laurer, Peter Rudolf, Dr.**
**Freinsheimer Strasse 33**
**D-6700 Ludwigshafen (DE)**
Erfinder: **Irgang, Matthias, Dr.**
**Gontardstrasse 4**
**D-6800 Mannheim (DE)**
Erfinder: **Pasedach, Heinrich, Dr.**
**Am Kirchberg 10**
**D-6719 Weisenheim am Berg (DE)**

**EP 0 008 741 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Verfahren zur Herstellung von Carbonylverbindungen

Die vorliegende Erfindung betrifft ein Verfahren zur Herstellung von Carbonylverbindungen der allgemeinen Formel I

$$R^1-\overset{\overset{\large R^2}{|}}{CH}-\overset{\overset{\large R^3}{|}}{CH}-\overset{\overset{\large R^4}{|}}{C}=O \qquad I,$$

in der $R^1$ Wasserstoff oder einen organischen Rest und $R^2$, $R^3$ und $R^4$ Wasserstoff oder eine $C_1$-$C_4$-Alkylgruppe bedeuten, durch selektive Hydrierung der α,β-ungesättigten Carbonylverbindung II

$$R^5-\overset{\overset{\large R^2}{|}}{C}=\overset{\overset{\large R^3}{|}}{C}-\overset{\overset{\large R^4}{|}}{C}=O \qquad II,$$

in der $R^5$ den gewünschten Rest $R^1$ oder einen Rest, der eine oder mehrere konjugierte Doppelbindungen in Konjugation zur $(-R^2C=CR^3-)$-Gruppierung enthält, bedeutet, mit Wasserstoff in Gegenwart von Palladiumkatalysatoren und organischen Basen in der Flüssigphase.

Speziell betrifft die Erfindung die selektive Hydrierung von Citral (IIa)

$$CH_3-\overset{\overset{\large CH_3}{|}}{C}=CH-CH_2-CH_2-\overset{\overset{\large CH_3}{|}}{C}=CH-CHO \qquad (IIa)$$

zu Citronellal (Ia)

$$CH_3-\overset{\overset{\large CH_3}{|}}{C}=CH-CH_2-CH_2-\overset{\overset{\large CH_3}{|}}{CH}-CH_2-CHO \qquad Ia.$$

Aus der DE-AS 21 14 211 ist es bekannt, Citronellal (Ia) durch Hydrierung von Citral (IIa) mit Wasserstoff in Gegenwart eines feuchten Palladiumkatalysators sowie einer Base in der Flüssigphase herzustellen. Als Basen werden auch organische Basen wie tertiäre Amine empfohlen, und zwar in einer Menge von 0,1 bis 10 Gew.% des Citrals.

Unbefriedigend bei diesem Verfahren sind indes die (jeweils in Relation zum angewandten Druck), verhältnismäßig langen Reaktionszeiten. Zwar können die Reaktionszeiten durch Verwendung größerer Mengen des Pd-Katalysators verkürzt werden, jedoch ist dies wieder mit dem Nachteil erhöhter Katalysatorkosten verbunden. Wenn der Katalysator auch nicht verbraucht wird, so wird er doch im Laufe der Zeit desaktiviert. Eine erfolgreiche Regenerierung des Katalysators ist jedoch im allgemeinen nicht möglich. Auch verfahrenstechnisch sind größere Katalysatormengen im Hinblick auf die Probleme der Handhabung von Feststoffen nachteilig.

Ähnlich verhält es sich mit dem Verfahren der DE-OS 25 34 747 nach welchem Citral oder seine Homologen in wäßrig-alkoholischem Medium sowie in Gegenwart einer Base selektiv zum Citronellal bzw. dessen Homologen hydriert werden sollenverwendet man hier anorganische Basen oder Trimethylamin in den empfohlenen relativ geringen Mengen, so sind die Reaktionszeiten in Relation zum angewandten Druck und der Katalysatormenge unbefriedigend oder man erhält unerwünscht hohe Mengen von 3,7-Dimethyloctanal bzw. dessen Homologen.

Nach dem Verfahren der FR-PS 2 224 433, welches ebenfalls die technisch vielbearbeitete selektive Hydrierung des Citrals und seiner Homologen zu den entsprechenden Citronellal-Verbindungen betrifft, soll diese in alkoholischem Medium durchzuführende Reaktion durch Mitverwendung von Borax sowie von Chinolin verbessert werden. Unter den im Beispiel angegebenen ungünstigen Verfahrensbedingungen — relativ große Katalysatormengen und unüblich geringer $H_2$-Druck (0,08 bar) — nimmt diese Umsetzung 37 Stunden in Anspruch, ohne daß man hier hinsichtlich der Dimethyloctanal-Bildung (1,2 %) ein besonders gutes Ergebnis erzielt.

Der Erfindung lag daher die Aufgabe zugrunde, die α,β-ungesättigten Carbonylverbindungen (II),

insbesondere das Citral (IIa) auf insgesamt wirtschaftlichere Weise als bisher zu den entsprechenden α,β-gesättigten Carbonylverbindungen (I) zu hydrieren.

Dementsprechend wurde ein verbessertes Verfahren zur Herstellung der Carbonylverbindungen (I) durch selektive Hydrierung der Carbonylverbindungen (II) mit Wasserstoff in Gegenwart von Palladiumkatalysatoren und organischen Basen in der Flüssigphase gefunden, welches dadurch gekennzeichnet ist, daß man die Hydrierung in Gegenwart von 15 bis 50 Gew.%, bezogen auf (II), eines tertiären Amins vornimmt.

Die erfindungsgemäße Maßnahme gestattet bei verbessertem Verfahrenserfolg eine erhebliche Einsparung an dem Pd-Katalysator, verglichen mit der herkömmlichen Arbeitsweise unter Verwendung geringer Mengen eines basischen Mittels wie eines tertiären Amins. Außerdem wird die Selektivität bezüglich der Verfahrensprodukte (I) verbessert, was vor allem für Duft- und Aromastoffe, z.B. das Citronellal, von Bedeutung ist, denn bei einer mit wirtschaftlich vertretbaren Aufwand vorgenommenen Reindarstellung des Produktes sinkt die Ausbeute um ein Mehrfaches als der Menge der Begleitstoffe entspricht.

Prinzipiell sind nach den bisherigen Beobachtungen jegliche tertiäre Amine geeignet, so daß es auf deren chemische Natur, sofern sie aufgrund funktioneller Gruppen nicht anderweitig mit den Reaktionspartner reagieren können, nicht ankommt. Genannt seien beispielsweise

— aliphatische tertiäre Amine mit insgesamt 3 bis 30 C-Atomen wie vor allem Trimethylamin sowie Triäthylamin, Triäthanolamin und Trihexylamin,
— cyclische tertiäre Amine wie N-Methylpiperidin, N-Methylmorpholin und N,N'-Dimethylpiperazin
— aliphatisch-cycloaliphatische tertiäre Amine wie N,N-Dimethylcyclohexylamin
— aliphatisch-araliphatische tertiäre Amine wie N,N-dimethylbenzylamin
— aliphatisch-aromatische tertiäre Amine wie N,N-Dimethylanilin sowie
— heterocyclisch-aromatische tertiäre Amine wie Pyridin und Chinolin.

Aus wirtschaftlichen Gründen wird man im übrigen möglichst billige Amine verwenden, deren Siedepunkt entweder deutlich tiefer oder deutlich höher ist, als der des Verfahrensproduktes, da sich in diesen Fällen entweder die Amine oder die Verfahrensprodukte leicht aus dem Reaktionsgemisch abdestillieren lassen.

Die Menge der Amine beträgt vorzugsweise 25 bis 40 Gew.% des Einsatzstoffes (II).

Als Hydrierkatalysatoren können alle handelsüblichen Palladiumkatalysatoren, oder Palladium-Trägerkatalysatoren, vor allem solche mit Kohle in Form von Russ oder Aktivkohle als Trägermaterial, verwendet werden. Besonders gut geeignet sind Aktivkohle-Trägerkatalysatoren mit einer Oberfläche von 600 bis 1 000 $m^2$/kg und einem häufigsten Porendurchmesser von 4 bis 50 Angström sowie einer mittleren Pd-Teilchengröße von 8 bis 50 Angström. Der Pd-Gehalt der Katalysatoren liegt zwischen 0,1 bis 50, vorzugsweise 0,5 und 20 Gew.%.

Je nach Art der Ausgangsstoffe (II) und den Reaktionsbedingungen sowie dem Pd-Gehalt und den physikalischen Eigenschaften der Katalysatoren benötigt man diese in Mengen von 0,01 bis 10 Gew.% von (II). Bevorzugt werden die relativ geringen Mengen zwischen 0,01 und 1,0 Gew.% von (II), da in diesem Falle einer der wesentlichen Vorteile der erfindungsgemäßen Verfahrensverbesserung, nämlich die kürzere Reaktionszeit, besonders groß ist.

Ein weiterer Vorteil des Verfahrens liegt darin, daß die sonst wegen der Selektivitätserhöhung empfohlene Mitverwendung von Wasser entbehrlich wird, wenngleich andererseits Wasser nicht stört, sondern in größerer Menge als 5 Gew.% des Reaktionsgemisches die Hydrierung lediglich verlangsamt.

Hingegen empfiehlt es sich, die Reaktion in Gegenwart eines Lösungsmittels vorzunehmen. Die Mengen an Lösungsmittel, liegen im allgemeinen zwischen 10 und 300, vorzugsweise 25 und 150 Gew.% von (II). Als Lösungsmittel eignen sich alle inerten Flüssigkeiten, in denen sowohl (I) und (II) als auch das mitverwendete tertiäre Amin löslich sind. In Betracht kommen beispielsweise die tertiären Amine selber sowie auch zusätzlich Alkohole wie Methanol und Äthanol, Äther, Aceton und unter den Reaktionsbedingungen flüssige Kohlenwasserstoffe wie Hexan und Cyclohexan. Bevorzugt wird Methanol, und zwar vor allem, wenn man Trimethylamin als Base verwendet.

Im übrigen nimmt man die Hydrierung wie üblich vor, also bei Temperaturen zwischen 5 und 150 °C, bei Normaldruck, geringfügig erhöhtem Druck oder auch bei einem höheren Druck bis etwa 50, vorzugsweise 10 bar.

Besondere Bedeutung hat das erfindungsgemäße Verfahren für die wegen der Mehrfachhydrierung und der Isomerisierung bekanntermaßen technisch problematische Hydrierung von Citral (IIa) zu Citronellal (Ia).

Ebensogut ist das Verfahren aber auch auf die sonstigen definitionsgemäßen Verbindungen (II) anwendbar, wobei es meistens auch die genannten Vorteile über die bisher bekannten Verfahren bietet.

Die Reste $R^5$ in den Verbindungen (II) können prinzipiell beliebig sein. Enthalten diese Reste eine oder mehrere zur α,β-Doppelbindung konjugierte Doppelbindungen, so werden diese, was erwünscht sein kann, ebenfalls hydriert. Sind die Doppelbindungen hingegen, wie bei (IIa), isoliert, so werden sie in aller Regel nicht angegriffen. Das gleiche gilt für die überwiegende Zahl der Substituenten, da selbst die empfindlichen Formylgruppen intakt bleiben.

Beispiele für $R^5$ sind Alkyl- und Alkenylreste mit 1 bis 20 C-Atomen und aromatischen Reste wie die Phenylgruppe. Diese Reste können ihrerseits beispielsweise Alkylgruppen, Alkoxygruppen, Formylgruppen, Carbalkoxygruppen, Acylgruppen, Hydroxylgruppen, Carboxylgruppen, Nitrilgruppen, Aminogruppen und Halogen als Substituenten tragen.

Beispiel 1

Hydrierung von Citral

Je 152 g handelsübliches Citral (Citralgehalt rund 145 g) wurden in Gegenwart von 1,5 g eines Pd/Aktivkohle-Katalysators unter einem Wasserstoffdruck von 1,05 bar bei 20 °C in Gegenwart von a Gew.% Trimethylamin und b Gew.% Methanol, jeweils bezogen auf das Citral (145 g = 100 %) bis zum Ende der Wasserstoffaufnahme hydriert, welches nach t Stunden erreicht war.

Der Katalysator enthielt 5 Gew.% Pd, der mittlere Durchmesser der Pd-Teilchen betrug 45 Angström, der häufigste Porendurchmesser der Aktivkohle betrug 5 Angström und die Oberfläche 857 $m^2/g$.

Die destillativ unter Zuhilfenahme der Gaschromatographie ermittelten Verfahrensergebnisse sind der nachstehenden Tabelle zu entnehmen.

| Vers.Nr. | a (Gew.%) | b (Gew.%) | t (h) | Umsatz (%) | Selektivität (%) (bezogen auf Umatz) Citronellal | Dimethyloctanal | Rückstand (g) |
|---|---|---|---|---|---|---|---|
| zum Vergleich | | | | | | | |
| 1 | 1 | 2,5 | 43 | 65 | 62 | 0,5 | 3 |
| 2 | 2 | 5 | 31 | 100 | 92 | 1,3 | 3 |
| 3 | 4 | 10 | 24 | 100 | 95 | 1,7 | 3 |
| 4 | 8 | 20 | 21 | 100 | 95 | 1,7 | 3 |
| 5 | 10 | 25 | 20 | 100 | 95 | 1,7 | 3 |
| erfindungsgemäß | | | | | | | |
| 6 | 20 | 50 | 16 | 100 | 95 | 0,8 | 3 |
| 7 | 30 | 100 | 13 | 100 | 96 | 0,8 | 3 |

Wie man erkennt, verkürzt sich durch die erfindungsgemäße Maßnahme die Reaktionszeit erheblich, und außerdem verbessert sich die Selektivität.

Beispiel 2

Hydrierung von Citral

Je 152 g handelsübliches Citral (Citralgehalt rund 145 g) wurden in Gegenwart von 0,4 g eines Pd/Aktivkohle-Katalysators unter einem Wasserstoffdruck von 6 bar bei 40 °C in Gegenwart von a Gew.% Trimethylamin und b Gew.% Methanol, jeweils bezogen auf das Citral (145 g = 100 %), bis zum Ende der Wasserstoffaufnahme hydriert, welches nach t Stunden erreicht war.

Der Katalysator enthielt 5 Gew.% Pd, der mittlere Durchmesser der Pd-Teilchen betrug 45 Angström, der häufigste Porendurchmesser der Aktivkohle betrug 5 Angström und die Oberfläche 850 $m^2/g$.

Die analog Beispiel 1 ermittelten Verfahrensergebnisse sind der nachstehenden Tabelle zu entnehmen.

| Vers.Nr. | a (Gew.%) | b (Gew.%) | t (h) | Umsatz (%) | Selektivität (%) (bezogen auf Umsatz) Citronellal | Dimethyloctanal | Rückstand (g) |
|---|---|---|---|---|---|---|---|
| zum Vergleich | | | | | | | |
| 1 | 6 | 12 | 13 | 99 | 97 | 2 | 1,5 |
| 2 | 8 | 16 | 12 | 100 | 97 | 1 | 2,0 |
| erfindungsgemäß | | | | | | | |
| 3 | 30 | 60 | 8 | 100 | 97 | 0,5 | 1,5 |
| 4 | 40 | 80 | 6 | 100 | 97 | 0,4 | 1,5 |

Auch hier bewirkt der erfindungsgemäße Zusatz des Amins eine deutliche Senkung der Reaktionszeit. Eine derartige Senkung ist, unter sonst gleichen Bedingungen, jedoch mit nur 10 Gew.% Amin, nur mit etwa der 10-fachen Katalysatormenge möglich.

## Beispiele 3 bis 8

Herstellung verschiedener Carbonylverbindungen (I)

Je 145 g einer α,β-ungesättigten Carbonylverbindung II wurden mittels 5 g des Katalysators aus Beispiel 1 in Gegenwart von 20 Gew.% Trimethylamin und 40 Gew.% Methanol, bezogen auf die Menge von (II), bei 40 °C unter einem Wasserstoffdruck von 6 bar hydriert, bis nach t Stunden keine Wasserstoffaufnahme mehr erfolgte. Die Ergebnisse sind aus der nachstehenden Tabelle ersichtlich.

| Beispiel | Verbindung (II) | Umsatz (%) | t (h) | Verfahrensprodukt I | Selektivität (bezogen auf den Umsatz) (%) |
|---|---|---|---|---|---|
| 3 | 6-Methylhept-3-en-2-on | 100 | 7 | 6-Methylheptan-2-on | 96 |
| 4 | 2-Äthylhex-2-en-1-al | 100 | 6 | 2-Äthylhexan-1-al | 96 |
| 5 | 2-Methylpent-2-en-1-al | 99 | 6 | 2-Methylpentan-1-al | 97 |
| 6 | Crotonaldehyd | 99 | 8 | Butyraldehyd | 90 |
| 7 | 3-Methylbut-2-en-1-al | 100 | 8 | 3-Methylbutyraldehyd | 93 |
| 8 | p-tert.-Butylphenylmethacrolein | 84 | 5 | p-tert.-Butylphenylisobubyraldehyd | 86 |

## Beispiel 9

Hydrierung von Citral

152 g handelsübliches Citral (Citralgehalt ca. 145 g) wurden in Gegenwart von 0,2 g des Palladiumkatalysators aus Beispiel 1 und 25 Gew.% Triäthylamin (bezogen auf Citral) unter einem Wasserstoffdruck von 6 bar bei 50 °C hydriert, wobei nach 8 Stunden die Wasserstoffaufnahme zum Stillstand kam. Die übliche Aufarbeitung lieferte 145 g Citronellal mit einem Reingehalt von 97,4 % neben 0,5 % Dimethyloctanal. Die Ausbeute betrug 96,8 %.

**Ansprüche**

1. Verfahren zur Herstellung von Carbonylverbindungen der allgemeinen Formel

$$R^1-\overset{\overset{R^2}{|}}{C}H-\overset{\overset{R^3}{|}}{C}H-\overset{\overset{R^4}{|}}{C}=O \qquad I,$$

in der $R^1$ Wasserstoff oder einen organischen Rest und $R^2$, $R^3$ und $R^4$ Wasserstoff oder eine $C_1$-$C_4$-Alkylgruppe bedeuten, durch selektive Hydrierung der α,β-ungesättigten Carbonylverbindungen II

$$R^5-\overset{\overset{R^2}{|}}{C}=\overset{\overset{R^3}{|}}{C}-\overset{\overset{R^4}{|}}{C}=O \qquad II,$$

in der $R^5$ den gewünschten Rest $R^1$ oder einen Rest, der eine oder mehrere konjugierte Doppelbindungen in Konjugation zur ($-R^2C=CR^3-$)-Gruppierung enthält, bedeutet, mit Wasserstoff in Gegenwart von Palladiumkatalysatoren und organischen Basen in der Flüssigphase, dadurch gekenn-

zeichnet, daß man die Hydrierung in Gegenwart von 15 bis 50 Gew.%, bezogen auf (II), eines tertiären Amins vornimmt.

2. Verfahren nach Anspruch 1, dadurch gekennzeichnet, daß man als Katalysator einen Pd/Aktivkohle-Trägerkatalysator verwendet, der einen Pd-Gehalt von 0,1 bis 50 Gew.% hat, bei dem der mittlere durchmesser der Pd-Teilchen 8 bis 50 Angström beträgt, der häufigste Porendurchmesser zwischen 4 und 50 Angström liegt und bei dem die Aktivkohle eine Oberfläche von 600 bis 1 000 $m^2/kg$ hat.

3. Verfahren nach den Ansprüchen 1 und 2, dadurch gekennzeichnet, daß als tertiäres Amin Trimethylamin verwendet wird.

4. Verfahren nach den Ansprüchen 1 bis 3, dadurch gekennzeichnet, daß es auf die Hydrierung von Citral zu Citronellal angewendet wird.

**Claims**

1. A process for the preparation of a carbonyl compound of the general formula

$$R^1-\underset{R^2}{\overset{|}{CH}}-\underset{R^3}{\overset{|}{CH}}-\underset{R^4}{\overset{|}{C}}=O \qquad I,$$

where $R^1$ is hydrogen or an organic radical and $R^2$, $R^3$ and $R^4$ are hydrogen or $C_1$-$C_4$-alkyl, by selective hydrogenation of the α,β-unsaturated carbonyl compound II

$$R^5-\underset{R^2}{\overset{|}{C}}=\underset{R^3}{\overset{|}{C}}-\underset{R^4}{\overset{|}{C}}=O \qquad II,$$

where $R^5$ is the desired radical $R^1$ or a radical containing one or more double bonds conjugated with the $(-R^2C{=}CR^3-)$ group, with hydrogen in the presence of a palladium catalyst and an organic base, in the liquid phase, characterized in that the hydrogenation is carried out in the presence of from 15 to 50 % by weight, based on (II), of a tertiary amine.

2. A process as claimed in claim 1, characterized in that the catalyst used is a Pd/active charcoal supported catalyst which has a Pd content of from 0.1 to 50 % by weight, a mean diameter of the Pd particles of from 8 to 50 Angström, a pore diameter distribution maximum at from 4 to 50 Angström, and a surface area, of the active charcoal, of from 600 to 1 000 $m^2/kg$.

3. A process as claimed in claims 1 and 2, characterized in that trimethylamine is used as the tertiary amine.

4. A process as claimed in claims 1 to 3, characterized in that it is used for the hydrogenation of citral to citronellal.

**Revendications**

1. Procédé pour la préparation de composés carbonylés de la formule générale

$$R^1-\underset{R^2}{\overset{|}{CH}}-\underset{R^3}{\overset{|}{CH}}-\underset{R^4}{\overset{|}{C}}=O \qquad I,$$

dans laquelle $R^1$ représente un atome d'hydrogène ou un reste organique et $R^2$, $R^3$ et $R^4$ désignent chacun un atome d'hydrogène ou un radical alcoyle en $C_1$ à $C_4$, par hydrogénation sélective de composés carbonylés à insaturation α, β de la formule

$$R^5-\underset{R^2}{\overset{|}{C}}=\underset{R^3}{\overset{|}{C}}-\underset{R^4}{\overset{|}{C}}=O \qquad II,$$

dans laquelle $R^5$ représente, soit le groupe $R^1$ voulu, soit un groupe contenant une ou plusieurs doubles liaisons conjuguées par rapport au groupement $- R^2 C = C R^3 -$, par l'hydrogène en phase liquide en présence d'un catalyseur au palladium et de bases organiques, caractérisé en ce que l'hydrogène en phase liquide en présence d'un catalyseur au palladium et de bases organiques, caractérisé en ce que l'hydrogénation est réalisée en présence de 15 à 50 % en poids, par rapport au composé de la formule II, d'une amine tertiaire.

2. Procédé suivant la revendication 1, caractérisé en ce que le catalyseur est un catalyseur au palladium sur support de charbon activé, dont la teneur en palladium est comprise entre 0,1 et 50 % en poids, dont les particules de palladium possèdent un diamètre moyen de 8 à 50 Angstrøms, la majorité des pores possédant un diamètre compris entre 4 et 50 Angstrøms et le charbon activé une surface de 600 à 1 000 $m^2$/kg.

3. Procédé suivant l'une des revendications 1 et 2, caractérisé en ce que l'amine tertiaire est la triméthylamine.

4. Procédé suivant l'une des revendications 1 à 3, caractérisé en ce qu'il est mis en œuvre pour l'hydrogénation du citral en citronellal.